# EUROPÄISCHE PATENTANMELDUNG

(11) **EP 1 777 287 A1**
(43) Veröffentlichungstag der Anmeldung: **25.04.2007**
(21) Anmeldenummer: 05109860.6
(22) Anmeldetag: 21.10.2005
(51) Int. Cl.: C11B 9/00, A23L 3/34

(54) **Allergiereduktion in Parfümölen und Duftstoffen**

(71) Anmelder: Symrise GmbH & Co. KG, 37603 Holzminden (DE)
(72) Erfinder: Panten, Johannes, 37671 Höxter (DE); Bertram, Heinz-Jürgen, 37603 Holzminden (DE)
(74) Vertreter: Eisenführ, Speiser & Partner

(57) **Zusammenfassung**

Die vorliegende Erfindung betrifft Systeme zum Schützen einer oxidationsempfindlichen Substanz, insbesondere eines Riech- und/oder Aromastoffs, vor Oxidation und insbesondere vor dem Ausbilden allergener Zersetzungsprodukte.

## Beschreibung

Die vorliegende Erfindung betrifft Systeme zum Schützen einer oxidationsempfindlichen Substanz, insbesondere eines Riech- und/oder Aromastoffs, vor Oxidation und insbesondere vor dem Ausbilden allergener Zersetzungsprodukte.

Riechstoffe und Parfümöle spielen eine bedeutende Rolle im täglichen Leben der Menschen. Heute erstreckt sich ihr Anwendungsgebiet auf fast alle Bereiche, vornehmlich jedoch Körperpflege- und Haushaltsprodukte. Seit kurzem geht man davon aus, dass einige Riechstoffe für allergische Reaktionen der menschlichen Haut ursächlich oder zumindest mitverantwortlich sind. Man vermutet eine Haptenisierung von Riechstoffen durch die Proteine der oberen Hautschicht. Diese "Allergische Kontakt-Dermatitis" durch Riechstoffe fällt in den sog. Allergie-Typ IV, eine durch T-Zellen vermittelte verzögerte Überempfindlichkeitsreaktion. Die Entwicklung dieses Typs läuft in zwei verschiedenen Phasen ab: in der Induktionsphase dringt ein kleines Molekül durch die Corneum-Schicht der Haut in die Epidermis ein und bindet dort an oder reagiert mit Hautproteinen. Dieser Komplex wird durch antigenpräsentierende Zellen der Haut (wie die Langerhans-Zellen) aufgenommen, bearbeitet und zusammen mit Klasse II MHC-Molekülen den T-Lymphozyten präsentiert. Die dadurch aktivierten T-Zellen teilen sich und differenzieren in sensibilisierte T-Zellen und Gedächtniszellen. In der Expressionsphase aktiviert eine folgende Aussetzung desselben Duftstoffes die sensibilisierten T Zellen, und bewirkt die Freisetzung von verschiedenartigen Cytokinen (beispielsweise IL-2, IFN-γ, MIF, TNF-β). Die Cytokine verursachen eine Ansammlung und Aktivierung von Makrophagen. Die Makrophagen setzen Enzyme frei, die eine lokalisierte Gewebe-Zerstörung verursachen.

Besonders Riechstoffe, die eine α,β-ungesättigte Carbonylfunktion aufweisen, neigen zu Haptenisierungsreaktionen, insbesondere mit Proteinen, die Aminosäuren mit freien Amino- oder Thiolgruppen enthalten, da Riechstoffe leicht über eine nucleophile 1,4-Addition an diese Gruppen binden können. Aber auch solche Riechstoffe, die z.B. durch radikalische Oxidation mit Luftsauerstoff ungesättigte Strukturen bilden können, sind häufig allergen. Dabei werden in einem Primärschritt zunächst Hydroperoxide gebildet. Diese können dann zu den ungesättigten Carbonylverbindungen zerfallen. Gewöhnlich werden bei Einsatz von oxidationsempfindlichen Stoffen Radikalfänger bzw. Antioxidantien zugesetzt. Diese Substanzen sind jedoch nicht in der Lage, bereits gebildete Peroxidstrukturen zu zerstören bzw. abzubauen.

Besonders bedeutsame oxidationsempfindliche Riechstoffe, die zu allergieauslösenden Strukturen oxidiert werden können, sind z.B.:
Limonen: 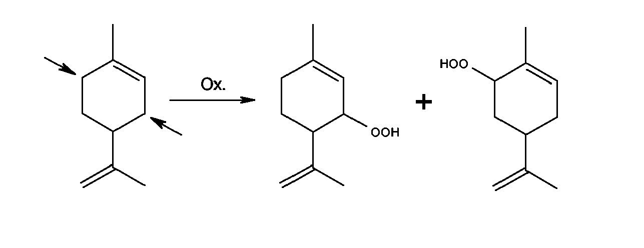
Geraniol: 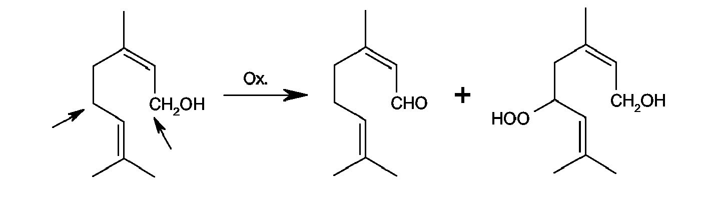
Citronellol:

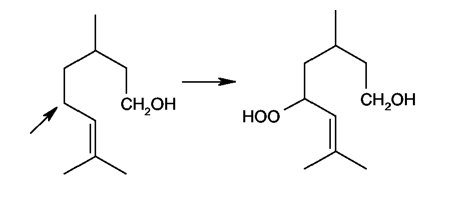

Eine besondere mechanistische Bedeutung kommt dabei den oben schon erwähnten Hydroperoxiden zu (s. Bild). Diese werden in einem ersten Schritt durch Insertion von Sauerstoffdiradikalen in aktivierte Allylpositionen (s. Pfeile) gebildet. Beim anschließenden Zerfall dieser Hydroperoxide können über einen Radikalkettenmechanismus beschleunigte Autoxidationen in Gang gesetzt werden. Eine besondere Aufgabe dieser Erfindung bestand also darin, ein System bereitzustellen, die einen Zerfall der Hydroperoxide ohne gleichzeitige Bildung weiterer Radikale und damit weiterer Autoxidationsprodukte bewirken.

Es ist bekannt, organische Peroxide mit bestimmten Materialien zu zerstören. So ist z.B. in US 3,884,836 die Wirkung eines geträgerten Metalloxidgemisches bestehend aus den Oxiden des Mangan, Cobalt, Kupfers, Silbers und Blei für die Darstellung von Sauerstoff aus H₂O₂ beschrieben. Als Trägermaterial dienen keramische Verbindungen wie Aluminiumsilicat, die Poren enthalten, in die sich die Oxide einlagern können.

Auch in JP 10330236 werden geträgerte Metalloxide und ihre Eigenschaft zum Einsatz gegen Peroxidbildung beschrieben.

Weiterhin sind aus JP 2004268968 werden funktionale Glascontainer bekannt, die an ihrer Innenseite mit schwarzem Kohlenstoff bzw. Tourmalin beschichtet sind. Beschrieben wird ihre Anwendung in Flüssigkeiten aus dem Food-Bereich, wie z.B. Beverages, um Redoxprozesse zu unterbinden.

Der Einsatz von Metalloxid-Nanopigmenten in Verbindung mit Polymeren wird in EP 0966954 als kosmetisches Sonnenschutzmittel erwähnt.

Desweiteren wird in Patent FR 2708851 der Einsatz eines Gemisches von Nanopigmenten aus Metalloxiden und Antioxidationsmitteln in kosmetischen Kompositionen beschrieben.

In Catalysis Today 1997, 36, 153-166 wird über die Eigenschaften von geträgerten Goldkatalysatoren berichtet. Es gibt kein Hinweis auf Peroxidspaltungen.

### Auch Reaktionen von ungeträgerten Metallen finden sich in der Literatur:

In JP 11-060493 wird die Wirkung von kolloidalem Platin und Palladium zur Vorbeugung von Krankheiten, die durch aktiven Sauerstoff ausgelöst werden, beschrieben.

In JP 11-346715 werden die gleichen Verbindungen wie in JP 11-060493 als Antioxidationsmittel in Food Produkten (s.o.) beschrieben.

Der Einsatz von homogenen Metallverbindungen zur Zerstörung von Peroxiden wird in DE 100 41 510 A1 am Beispiel von homogenen Gold- und Silberverbindungen beschrieben.

Ein weiteres Beispiel sind cyclische Mangan-Salenverbindungen in WO 02/44187, die als Antioxidansmittel auch in Kosmetika erwähnt werden.

Es ist auch bekannt, Materialien mit oberflächenaktiven Substanzen zu beschichten, um gewisse Redoxreaktionen hervorzurufen. Als Beschichtungstechniken bieten sich vornehmlich das Aufsintern von oxidischen Materialien und die reduktive Fällung von Metallen aus Lösungen an.

Ein Nachteil der bekannten Dotierung der Glascontainer mit schwarzem Kohlenstoff besteht darin, dass sie dadurch keinen Blick mehr in das Innere eines Glasgefäßes zulassen, was zumindest bei den in der Parfümerie verwendeten Glasflacons wünschenswert ist.

Ein weiterer Nachteil der beschriebenen homogenen Metallverbindungen besteht darin, dass sie aus einer Lösung nur sehr schwer abzutrennen sind.

Der größte Nachteil der beschriebenen Systeme besteht jedoch darin, dass alle Materialien sowohl die Peroxidzerstörung, als auch wieder die Peroxidbildung katalysieren können, da es sich in den meisten Fällen um eine reversible Reaktion handelt.

Es war daher eine Aufgabe der vorliegenden Erfindung, ein System bereitzustellen zum Schützen einer oxidationsempfindlichen Substanz, insbesondere eines Riechtstoffs und/oder Aromastoffs, vor Oxidation. Das System sollte es nach Möglichkeit erlauben, das allergene Potential zu verringern, das sich bei der Lagerung von oxidationsempfindlichen Riechstoffen entwickelt, so dass nach Möglichkeit von den gelagerten Riechstoffen keine signifikante allergene Wirkung mehr ausgeht. Eine weitere Aufgabe war es, entsprechend behandelte Riechstoffzubereitungen, kosmetische und/oder dermatologische Produkte anzugeben.

Erfindungsgemäß wird deshalb ein System angegeben zum Schützen einer oxidationsempfindlichen Substanz, umfassend
(a) eine Oberfläche, die ein oder mehrere Metalle der Gruppe Mangan, Cobalt, Kupfer, Silber, Gold, Palladium, Platin und Blei und/oder dessen bzw. deren Oxide enthält, und ferner
(b) ein oder mehrere Antioxidantien.

Überraschenderweise hat sich nunmehr herausgestellt, dass die Metalle und Metalloxide gemäß (a) mit Antioxidantien vorteilhaft zusammenwirken zum Schützen oxidationsempfindlicher Substanzen vor Oxidation.

Das erfindungsgemäße System kann insbesondere ausgebildet sein als ein Gefäß mit einem Innenraum, in dem das oder die Antioxidantien gemäß (b) sowie die metall- und/oder metalloxidhaltige Oberfläche gemäß (a) vorliegen, wobei die Oberfläche insbesondere ein Teil oder die Gesamtheit der Oberfläche des Gefäß-Innenraums sein kann, oder ein Teil oder die Gesamtheit der Oberfläche eines Füllkörpers ausmachen kann. Das erfindungsgemäße System kann jedoch auch in Form einer Zubereitung der zu schützenden Substanz vorliegen, bei der die zu schützende Substanz (insbesondere ein Riechstoff und/oder ein Aromastoff) gemischt ist mit einem oder mehreren Antioxidantien gemäß (b) und Körpern mit einer Oberfläche gemäß (a).

Besonders bevorzugt ist ein solches erfindungsgemäßes System, dessen Oberfläche gemäß (a) eine Oberfläche in einem Innenraum eines Gefäßes zum Aufbewahren einer oxidationsempfindlichen Substanz ist, und der Innenraum ferner das oder die Antioxidantien gemäß (b) enthält.

Im Sinne dieser Erfindung werden insbesondere solche Substanzen, insbesondere Riechstoffe und Aromastoffe, als oxidationsempfindlich bezeichnet, die eine ungesättigte Bindung in α-Stellung zu einem durch das erfindungsgemäße System vor Oxidation zu schützenden Kohlenstoffatom aufweisen. Dies sind insbesondere jene Stoffe, die bei Oxidation an Luft eine α,β-ungesättigte Carbonylfunktion ausbilden können. Dementsprechend wird durch Einsatz eines erfindungsgemäßen Systems auf vorteilhaft einfache Weise die Ausbildung allergener Zersetzungsprodukte bei der Lagerung einer oxidationsempfindlichen Substanz unterdrückt, insbesondere bei der Lagerung eines Riech- und/oder Aromastoffs. Das erfindungsgemäße System ist besonders gut geeignet, die oxidationsempfindlichen und bevorzugten Substanzen Limonen, Geraniol und Citronellol zu schützen.

Das erfindungsgemäße System ist insbesondere geeignet zum Verringern der Peroxidzahl einer Zubereitung enthaltend eine oxidationsempfindliche Substanz. Die Peroxidzahl wird dabei bestimmt gemäß ISO-Standard 3960/TC.

Die Metalle und Metalloxide gemäß (a) sind auf einer Trägerstoff-Oberfläche angeordnet. Als Trägerstoffe kommen alle Materialien in Frage, die sich zu den bestimmungsgemäßen Anwendungen des erfindungsgemäßen Systems inert verhalten bzw. sich nur mit den Autoxidationsprodukten umsetzen. In einschlägigen Lehrbüchern werden diese Materialien grob als "anorganische Werkstoffe" bezeichnet. Sie beinhalten alle Formen von Glas, Keramiken, Porzellan, Stein, Minerale (natürliche und synthetische) und Bindemittel (Gips, Anhydrit, Kalk, Wasserglas, Zement etc.). Die Oberfläche des Trägerstoffs ist zweckmäßigerweise in der Lage, Metalle und/oder Metalloxide zu binden. Der Begriff Bindung bezeichnet in diesem Falle eine physikalische Bindung. Weder der Trägerstoff noch die geschichteten Metalle und Metalloxide verlieren bei der Bindung der Metalle bzw. Metalloxide ihre chemischen Eigenschaften.

Als Metalle sind im erfindungsgemäßen System bevorzugt einsetzbar Übergangselemente wie Cu, Ag, Au, Pd oder Pt. Als Metalloxide eignen sich bevorzugt die Oxide der Nebengruppenelemente, besonders bevorzugt diejenigen des Mangans, Kupfers, Silbers, Zinks und Bleis. Alle Metalle und Metalloxide können gemischt mit weiteren der erfindungsgemäß eingesetzten Metallen und Metalloxiden auf der Oberfläche gemäß (a) gebunden vorliegen.

Als Metalle und Metalloxide gemäß (a) ist besonders Mangan und insbesondere Manganoxid bevorzugt. Vorzugsweise wird das Mangan, und insbesondere das bevorzugte Manganoxid, durch weitere Substanzen abriebfest auf der Trägerstoff-Oberfläche angeordnet. Hierzu besonders bevorzugt ist es, wenn Mangan und insbesondere Manganoxid als Metall bzw. Metalloxid gemäß (a) in Verbindung mit Metallen und Metalloxiden eingesetzt wird, wobei die Metalle und Metalloxide ausgewählt sind aus Cobalt, Kupfer und Silber und deren Oxiden und Mischungen der genannten Metalle und Metalloxide. Besonders bevorzugt ist daher das Metall und Metalloxid gemäß (a) eine Metalloxid-Mischung mit folgenden Metallgehalten: Mangan: 30 bis 65 Gew.-%; Cobalt 5 bis 15 Gew.-%; Kupfer 15 bis 37 Gew.-%; Silber 12 bis 30 Gew.-%. Herstellverfahren entsprechender Mischungen gemäß (a) und weitere bevorzugte Mischungen sind in der US 3,884,836 beschrieben, deren Inhalt zum Zwecke der Offenbarung der vorliegenden Erfindung vollständig in Bezug genommen wird. In der genannten US-Schrift sind Mischungen enthaltend Blei bzw. Bleioxid beschrieben; es ist erfindungsgemäß ferner bevorzugt, die Zusammensetzung der Mischung (von Metallen und Metalloxiden) gemäß (a) zu wählen wie die in der US-Schrift angegebenen Zusammensetzungen, nur ohne Blei und Bleioxid.

Antioxidationsmittel, die im Sinne der Erfindung kombiniert werden können, sind definiert als Substanzen, die in im Vergleich zu dem oxidierbaren Substrat (Riechstoff) kleinen Konzentrationen die Oxidation des Substrats signifikant verzögern oder gänzlich verhindern.

Einige natürliche und wichtige Antioxidantien sind komplexe Mischungen, beispielsweise Extrakte oder Fraktionen von Pflanzen wie z. B. Grüntee, Rooibos, Honeybush, Traube, Rosmarin, Salbei, Melisse, Thymian, Lavendel, Olive, Hafer, Kakao, Ginkgo, Ginseng, Süßholz, Geißblatt, Sophora, Pueraria, Pinus, Citrus, Phyllanthus emblica oder Johanniskraut. Ferner bevorzugte Antioxidationsmittel sind die Tocopherole (Vitamin E), L-Ascorbinsäure (Vitamin C) und Glutathion, sowie Ubichinol, β-Carotin und Bilirubin (Abbauprodukt von Porphyrin-Derivaten) (vgl. C.-M. Andersson, A. Hallberg, T. Högberg, "Advances in the Development of Pharmaceutical Antioxidants", in Adv. Drug Res., B. Testa, U.A. Meyer (Hrsg.), Academic Press, London, 1996, S. 65-180). Daneben sind noch die mehrwertigen Säuren wie z.B. Zitronensäure und Aminosäuren als Antioxidantien geeignet. Ein weiteres Antioxidans speziell in der Haut ist das in den Melanocyten gebildete Melanin, ein meist braun-schwarzes Polymer, dass durch Oxidation und Polymerisation aus aromatischen Aminosäuren wie L-Tyrosin gebildet wird (vgl. M.R. Chedekel, Cosmetics & Toiletries 1996, 111(1), 71-74).

Die wichtigsten nichtnatürlichen Antioxidantien, die vor allem in der Nahrungsmittelindustrie zur Stabilisierung von Fetten und Ölen eingesetzt werden, sind 2- bzw. 3-tert-Butyl-4-methoxyphenol (1:9-Gemisch, butyliertes Hydroxyanisol, BHA) und 2,6-Di-tert-butyl-4-methylphenol (butyliertes Hydroxytoluol, BHT), Gallussäurepropylester (PG), Gallussäuredodecanylester (DG) und 2-tert-Butyl-1,4-dihydroxybenzol (TBHQ). Einige dieser synthetischen Antioxidantien sind allerdings aus toxikologischer Sicht als bedenklich eingestuft worden (vgl. S.M. Barlow, "Toxicological Aspects of Antioxidants Used as Food Additives", in Food Antioxidants, B.J.F. Hudson (Hrsg.), Elsevier, London, 1990, S. 253-307).

Als Antioxidantien sind weiterhin besonders geeignet: Aminosäuren (z.B. Glycin, Histidin, 3,4-Dihydroxyphenylalanin, Tyrosin, Tryptophan) und deren Derivate, Imidazole (z.B. Urocaninsäure) und deren Derivate, Peptide (D,L-Carnosin, D-Carnosin, L-Carnosin, Anserin) und deren Derivate, Carotinoide, Carotine (z.B. α-Carotin, β-Carotin, Lycopin) und deren Derivate, Chlorogensäure und deren Derivate, Liponsäure und deren Derivate, Aurothioglucose, Propylthiouracil und andere Thiole (z.B. Thioredoxin, Glutathion, Cystein, Cystin, Cystamin und deren Glycosyl- und N-Acylderivate oder deren Alkylester) sowie deren Salze, Dilaurylthiodipropionat, Distearylthiodipropionat, Thiodipropionsäure und deren Derivate sowie Phenolsäureamide phenolischer Benzylamine (z.B. Homovanillinsäure-, 3,4-Dihydroxyphenylessigsäure-, Ferulasäure-, Sinapinsäure-, Kaffeesäure-, Dihydroferulasäure-, Dihydrokaffeesäure-, Vanillomandelsäure- oder 3,4-Dihydroxymandelsäureamide des 3,4-Dihydroxybenzyl-, 2,3,4-Trihydroxybenzyl- bzw. 3,4,5-Trihydroxybenzylamins), ferner (Metall-)chelatoren (z.B. 2-Hydroxyfettsäuren, Phytinsäure, Lactoferrin), Huminsäure, Gallensäuren, Gallenextrakte, Bilirubin, Biliverdin, Folsäure und deren Derivate, Ubichinon und Ubichinol und deren Derivate, Vitamin C und dessen Derivate (z.B. Ascorbylpalmitat, Magnesiumascorbylphosphat, Ascorbylacetat), Tocopherole und Derivate (z.B. Vitamin-E-Acetat), Vitamin A und Derivate (z.B. Vitamin-A-Palmitat), Rutinsäure und deren Derivate, Flavonoide (z.B. Quercetin, α-Glucosylrutin) und deren Derivate, Phenolsäuren (z.B. Gallussäure, Ferulasäure) und deren Derivate (z.B. Gallussäurepropylester, -ethylester, -octylester), Carnosolsäure und dessen Derivate, Rosmarinsäure und dessen Derivate, Furfurylidenglucitol, Dibutylhydroxytoluol, Butylhydroxyanisol, Harnsäure und deren Derivate, Mannose und deren Derivate, Zink und dessen Derivate (z.B. ZnO, ZnSO₄), Selen und dessen Derivate (z.B. Selenomethionin), Stilbene und deren Derivate (z.B. Stilbenoxid, Resveratrol) und weitere geeignete Derivate dieser genannten Wirkstoffe.

Um den angestrebten Effekt zu erreichen, ist es einerseits möglich, die Innenwände von Aufbewahrungs-/Reaktionsgefäßen im Sinne einer Oberfläche gemäß (a) zu belegen, andererseits können aber auch Füllkörper im Sinne einer Oberfläche gemäß (a) zubereitet werden. Möglichst grobe Füllkörper und insbesondere Glaskugeln mit einem Durchmesser, der vorzugsweise im Bereich von 2 bis 10 mm, bevorzugt im Bereich von 3 bis 8 mm liegt. Bevorzugt werden insbesondere Flint- oder Opalgläser bieten gegebenenfalls Vorteile in der Handhabung, da sie leicht von den zu schützenden Substanzen abtrennbar sind. Glaskugeln haben auch den Vorteil, zu optisch ansprechenden Gestaltungen ausformbar zu sein.

Das erfindungsgemäße System enthält das oder die Antioxidantien in einer wirksamen Menge, insbesondere zum Stabilisieren eines Riechstoffs und/oder Aromastoffs gegen Oxidation an Luft und/oder zum Verbessern der Lagerungsstabilität und/oder zum Verringern der allergenen Wirkung eines Riechstoffs, und gegebenenfalls andere Bestandteile. Das oder die Antioxidantien können dabei, insbesondere zusammen mit der zu schützenden Substanz, als "Wasser in Öl"-, "Öl in Wasser"-, "Wasser in Öl in Wasser"- oder "Öl in Wasser in Öl"-Emulsionen, als Mikroemulsionen, als Gele, als Lösungen z.B. in Ölen, Alkoholen oder Siliconölen vorliegen. Weitere übliche Hilfs- und Zusatzstoffe können in Mengen von 5 bis 99,9999 Gew.-%, vorzugsweise 10 bis 80 Gew.-%, bezogen auf das Gesamtgewicht der Formulierung, enthalten sein. Ferner können die Formulierungen Wasser in einer Menge bis zu 99,999 Gew.-%, vorzugsweise 5 bis 80 Gew.-%, bezogen auf das Gesamtgewicht der Formulierung, aufweisen.

Das oder die Antioxidantien können in einer weiteren bevorzugten Ausführungsform auch vor dem Mischen mit der oder den zu schützenden Substanz(en), insbesondere dem oder den Riechstoff(en) und/oder Aromastoff(en), in Liposomen, z.B. ausgehend von Phosphatidylcholin, in Microsphären, in Nanosphären oder auch in Kapseln aus einer geeigneten Matrix, z.B. aus natürlichen oder synthetischen Wachsen, beispielsweise Bienenwachs, Carnaubawachs, Siliconwachs oder Stearylalkohol, Eicosanol, Cetylalkohol, Stearin oder Paraffinwachs oder aus Gelatine, eingearbeitet werden. Eine weitere Ausführungsform besteht darin, dass die Antioxidantien vorher dem Mischen mit der oder den zu schützenden Substanz(en) mit Komplexbildnern, beispielsweise mit Cyclodextrinen oder Cyclodextrinderivaten, bevorzugt Methylcyclodextrin, komplexiert werden.

In einem bevorzugten System sind die zu schützende Substanz bzw. die zu schützenden Substanzen gemischt mit dem oder den Antioxidantien. Dabei macht das Antioxidans einen Anteil von vorzugsweise 0,001 bis 3 Gew.-% bezogen auf die gesamte Mischung aus. Besonders bevorzugt ist eine Mischung, die einen Gesamtanteil an Antioxidans von 0,1-2 Gew.-% bezogen auf die gesamte Mischung aufweist.

Das erfindungsgemäße System ist besonders geeignet zum Schützen einer Substanz und insbesondere eines Riech- oder Aromastoffs, die bzw. der eine α,β-ungesättigte Carbonylfunktion aufweist und/oder bei Oxidation an Luft eine α,β-ungesättigte Carbonylfunktion bildet. Derartige Substanzen, namentlich Riech- und Aromastoffe, haben ein besonders hohes allergenes Potential. Durch die Verwendung von mit Metallen oder Metalloxiden dotierten Glas- oder Keramikgegenständen in Kombination mit Antioxidantien lassen sich solche Substanzen, insbesondere Riech- und Aromastoffe, besonders gut gegen Oxidation stabilisieren, so dass eine derart stabilisierte Substanz (insbesondere ein Riechstoff bzw. ein Aromastoff) eine im Vergleich zur nicht mit dem erfindungsgemäß stabilisierten Substanz verringerte allergene Wirkung besitzt.

Das erfindungsgemäße System enthält das oder die Antioxidantien gemäß (b) vorzugsweise in einem kosmetischen und/oder dermatologischen Produkt. Das erfindungsgemäße System vermittelt diesem Produkt die oben beschriebenen Vorteile, insbesondere die verbesserte Stabilität gegen Oxidation an Luft und eine verringerte allergene Wirkung des Riechstoffs.

Die Menge der Antioxidantien (eine oder mehrere Verbindungen) in den erfindungsgemäßen kosmetischen und/oder dermatologischen Produkten bzw. Zubereitungen, beträgt vorzugsweise 0,0001 bis 3 Gew.-%, besonders bevorzugt 0,001 bis 1 Gew.-%, insbesondere bevorzugt 0,001 bis 0,2 Gew.-%, bezogen auf das Gesamtgewicht der Produkte.

Die kosmetischen und dermatologischen Zubereitungen können kosmetische Hilfs- und Zusatzstoffe enthalten, wie sie üblicherweise in solchen Zubereitungen vewendet werden, z.B. Sonnenschutzmittel (z.B. organische oder anorganische Lichtfiltersubstanzen, bevorzugt Mikropigmente), Konservierungsmittel, Bakterizide, Fungizide, Viruzide, Kühlwirkstoffe, Pflanzenextrakte, entzündungshemmende Wirkstoffe, die Wundheilung beschleunigende Stoffe (z.B. Chitin oder Chitosan und dessen Derivate), filmbildende Substanzen (z.B. Polyvinylpyrrolidone oder Chitosan oder dessen Derivate), Vitamine (z.B. Vitamin C und Derivate, Tocopherole und Derivate, Vitamin A und Derivate), 2-Hydroxycarbonsäuren (z.B. Citronensäure, Äpfelsäure, L-, D-, oder dl-Milchsäure), Hautaufhellungsmittel (z.B. Kojisäure, Hydrochinon oder Arbutin), Hautfärbungsmittel (z.B. Walnussextrakte oder Dihydroxyaceton), Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchthaltende Substanzen (z.B. Glycerin oder Harnstoff), Fette, Öle, ungesättigte Fettsäuren oder deren Derivate (z.B. Linolsäure, α-Linolensäure, γ-Linolensäure oder Arachidonsäure und deren jeweiligen natürlichen oder synthetischen Ester), Wachse oder andere übliche Bestandteile einer kosmetischen oder dermatologischen Formulierung wie Alkohole, Polyole, Polymere, Schaumstabilisatoren, Elektrolyte, organische Lösungsmittel, Silikonderivate oder Chelatbildner (z.B. Ethylendiamintetraessigsäure und Derivate). Die jeweils einzusetzenden Mengen an kosmetischen oder dermatologischen Hilfs- und Zusatzstoffen und Parfüm können in Abhängigkeit von der Art des jeweiligen Produkts vom Fachmann durch einfaches Ausprobieren leicht ermittelt werden.

Besonders bevorzugt können die kosmetischen und/oder dermatologischen Zubereitungen auch Wirkstoffe zur Hautaufhellung enthalten. Insbesondere können die erfindungsgemäßen topischen kosmetischen Mittel auch Benzaldoxime mit mindestens einer aromatischen Hydroxy- oder Alkoxygruppe, Kojisäure, Kojisäurederivate, Ascorbinsäure, Ascorbinsäurederivate, Hydrochinon, Hydrochinonderivate, schwefelhaltigen Moleküle (z.B. Glutathion oder Cystein) oder andere synthetische oder natürliche Wirkstoffe zur Hautaufhellung enthalten, wobei letztere auch in Form eines Extrakts aus Pflanzen (z.B. Tocopherole und Derivate, Arbutin (z.B. aus Bearberry-Extrakt), Aloesin (z.B. aus Aloe-Extrakt), Grapefruit-Extrakt und Reis-Extrakt) verwendet werden können.

Die Menge der vorgenannten beispielhaften Wirkstoffe zur Hautaufhellung (eine oder mehrere Verbindungen) kann in den kosmetischen oder dermatologischen Zubereitungen 0,001 bis 30 Gew.-%, bevorzugt 0,001 bis 20 Gew-%, besonders bevorzugt 0,001 bis 5 Gew.-%, bezogen auf das Gesamtgewicht der Zubereitung, betragen.

Besonders bevorzugt sind erfindungsgemäße Systeme mit solchen kosmetischen oder dermatologischen Zubereitungen, die gleichzeitig in Form eines Sonnenschutzmittels vorliegen. Diese enthalten neben einem wirksamen Anteil an einem oder mehreren Antioxidantien gemäß (b) und einer Oberfläche gemäß (a) auch Sonnenschutzsubstanzen, bevorzugt organische oder anorganische Lichtfiltersubstanzen, insbesondere Mikropigmente. Die Sonnenschutzmittel können aber auch UVA- und/oder UVB-Filtersubstanzen enthalten, wobei die Gesamtmenge an Filtersubstanzen 0,1 bis 30 Gew.-%, vorzugsweise 0,5 bis 10 Gew.-%, bezogen auf das Gesamtgewicht der Zubereitungen, betragen kann, wobei man Sonnenschutzmitteln für Haut und Haar erhält. Als UV-Filtersubstanzen können beispielsweise 3-Benzylidencampherderivate (z.B. 3-(4-Methylbenzyliden)-dl-campher), Aminobenzoesäurederivate (z.B. 4-(N,N-Dimethylamino)benzoesäure-2-ethylhexylester oder Menthylanthranilat), 4-Methoxycinnamate (z.B. 2-Ethylhexyl-p-methoxycinnamat oder Isoamyl-p-methoxycinnamat), Benzophenone (z.B. 2-Hydroxy-4-methoxybenzophenon), ein- oder mehrfach sulfonierte UV-Filter [z.B. 2-Phenylbenzimidazol-5-sulfonsäure, Sulisobenzone oder 1,4-Bis(benzimidazolyl)-benzol-4,4',6,6'-tetrasulfonsäure bzw. 3,3'-(1,4-Phenylendimethyliden)-bis-(7,7-dimethyl-2-oxo-bicyclo-[2,2,1]heptan-1-methansulfonsäure) und deren Salze], Salicylate (z.B. 2-Ethylhexylsalicylat oder Homomenthylsalicylat), Triazine {z.B. 2,4-Bis-[4-(2-ethylhexyloxy)-2-hydroxyphenyl]-6-(4-methoxyphenyl)-1,3,5-triazin, 4,4'-([6-([(1,1-dimethylethyl)-aminocarbonyl]phenylamino)-1,3,5-triazin-2,4-diyl]diimino)bisbenzoesäurebis-(2-ethylhexyl)-ester)}, 2-Cyanopropensäurederivate (z.B. 2-Ethylhexyl-2-cyano-3,3-diphenyl-2-propenoat), Dibenzoylderivate (z.B. 4-tert-Butyl-4'-methoxydibenzoylmethan), polymergebundende UV-Filter (z.B. Polymer von N-[2-(bzw. 4)-(2-Oxo-3-bornyliden)methyl]benzylacrylamid) oder Pigmente (z.B. Titandioxide, Zirkondioxide, Eisenoxide, Siliciumdioxide, Manganoxide, Aluminiumoxide, Ceroxide oder Zinkoxide) verwendet werden.

Die Lipidphase in den kosmetischen oder dermatologischen Zubereitungen kann vorteilhaft gewählt werden aus folgenden Substanzgruppen: Mineralöle (vorteilhaft Paraffinöl), Mineralwachse, Kohlenwasserstoffe (vorteilhaft Squalan oder Squalen), synthetische oder halbsynthetische Triglyceridöle (z.B. Triglyceride der Caprin- oder Caprylsäure), natürliche Öle (z.B. Rizinusöl, Olivenöl, Sonnenblumenöl, Sojaöl, Erdnussöl, Rapsöl, Mandelöl, Palmöl, Kokusöl, Palmkernöl, Borretschsamenöl und dergleichen mehr), natürliche Esteröle (z.B. Jojobaöl), synthetische Esteröle (bevorzugt Ester von gesättigten und/oder ungesättigten, linearen und/oder verzweigten Alkancarbonsäuren von 3 bis 30 C-Atomen mit gesättigten und/oder ungesättigten, linearen und/oder verzweigten Alkoholen mit 3 bis 30 C-Atomen und Ester von aromatischen Carbonsäuren mit gesättigten und/oder ungesättigten, linearen und/oder verzweigten Alkoholen mit 3 bis 30 C-Atomen, insbesondere ausgewählt aus der Gruppe Isopropylmyristat, Isopropylstearat, Isopropylpalmitat, Isopropyloleat, n-Butylstearat, n-Hexyllaurat, n-Decyllaurat, Isooctylstearat, Isononylstearat, Isononylisononanoat, 2-Ethylhexylpalmitat, 2-Ethylhexyllaureat, 2-Hexyldecylstearat, 2-Octyldecylpalmitat, Oleyloleat, Oleylerucat, Erucyloleat, Erucylerucat sowie synthetische oder natürliche Gemische solcher Ester), Fette, Wachse und andere natürliche und synthetische Fettkörper, vorzugsweise Ester von Fettalkoholen mit Alkoholen niedriger C-Zahl (z.B. mit Isopropanol, Propylenglycol oder Glycerin) oder Ester von Fettalkoholen mit Alkansäuren niedriger C-Zahl oder mit Fettsäuren, Alkylbenzoate (z.B. Gemische von n-Dodecyl-, n-Tridecyl-, n-Tetradecyl- und n-Pentadecylbenzoat) sowie cyclische oder lineare Silikonöle (wie z.B. Dimethylpolysiloxane, Diethylpolysiloxane, Diphenylpolysiloxane sowie Mischformen daraus).

Die wässrige Phase der im erfindungsgemäßen System vorteilhaft eingesetzten kosmetischen oder dermatologischen Zubereitungen enthält gegebenenfalls bevorzugt Alkohole, Diole oder Polyole niedriger C-Zahl, sowie deren Ether, vorzugsweise Ethanol, Isopropanol, Propylenglycol, Glycerin, Ethylenglycol, Ethylenglycolmonoethyl- oder -monobutylether, Propylenglycolmonomethylether-, -monoethyl- oder -monobutyl-ether, Diethylenglycolmonomethyl- oder monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft ausgewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, jeweils einzeln oder in Kombination oder aus der Gruppe der Polyurethane.

Insbesondere bevorzugt ist die Verwendung der erfindungsgemäßen Zubereitungen zum Schutz von Geweben und Zellen von Säugern vor potentiell allergenen Duftstoffen. Die erfindungsgemäßen kosmetischen oder dermatologischen Zubereitungen dienen vor allem dem Schutz der Haut. Die Zubereitungen, in denen sie enthalten sind, greifen die Haut weniger an als diejenigen, in denen sie nicht enthalten sind, insofern schützen die Zubereitungen die Haut dadurch, dass sie weniger allergieauslösende Stoffe enthalten.

Die nachfolgend genannten Beispiele demonstrieren in anschaulicher Weise die oxidationsschützende, peroxidzersetzende Wirkung erfindungsgemäß beschichteter Glasgegenstände in Kombination mit Antioxidantien:

### Beispiel 1:

20 g Citronellol werden 72 h in einem Oxi-press gerät der Fa. Mikrolab A/S (Aarhus, Dänemark) mit Sauerstoff oxidiert (Bedingungen: Sauerstoffdruck: 5 bar, Temperatur: 60°C, Volumen des Autoklaven: 100ml). Dabei steigt die naßchemisch gemessene Peroxidzahl (Methode nach ISO-Standard 3960 / TC (Technical Comitee) 54) von 11 ml/g auf 102 ml/g an. Auch ein GC/MS zeigt, dass Peroxide entstanden sind. Diese oxidierte Probe wird anschließend für folgende Versuche eingesetzt:
a) 4 g der oxidierten Probe werden in ein Glasgefäß (2,7cm Durchmesser, 5cm Höhe), dessen Oberfläche mit Manganoxid beschichtet war, gegeben, mit 1000 ppm Rosmarinextrakt versetzt und bei Raumtemperatur stehengelassen. Nach 24 h waren 15 % der Peroxide, nach 72 h weitere 20 % der Peroxide abgebaut. Bei Behandlung desgleichen oxidierten Musters nur mit Manganoxid, ohne Rosmarinextrakt, stieg der Peroxidwert nach 48 h wieder auf den Anfangswert an.
b) 4 g der oxidierten Probe wurden in ein Glasgefäß (s.o.) gegeben, in dem sich eine Glaskugel befand, die auf ihrer Oberfläche mit einem Gemisch der Oxide der Metalle Mn, Co, Cu und Ag beschichtet war. Anschließend wurde nach Zugabe von 1000 ppm Rosmarinextrakt wieder stehengelassen.

| | |
|---|---|
| Peroxidabbau: | 24 h 8 % |
| | 72 h 30 % |

Die mit den beschichteten Gläsern behandelten Peroxide wurden anschließend analysiert. Dabei zeigten die GC/MS-Auswertungen, dass keine signifikante Menge der allergen wirkenden α,β-ungesättigten Carbonylgruppen entstanden ist. Zum Nachweis einer geringeren allergenen Wirkung wurden die so behandelten Proben anschließend mit einer in-vitro Prescreening-Methode untersucht. Dabei wird die Reaktionsfähigkeit von speziellen Aminosäuren mit den Duftstoffen als Maß für deren allergenes Potential herangezogen. Auch hier zeigte sich, dass durch die Behandlung das allergene Potential herabgesetzt wird.

## Patentansprüche

1. System zum Schützen einer oxidationsempfindlichen Substanz, umfassend
(a) eine Oberfläche aufweist, die ein oder mehrere Metalle der Gruppe Mangan, Cobalt, Kupfer, Silber, Gold, Palladium, Platin und Blei und/oder dessen bzw. deren Oxide enthält, und ferner
(b) ein oder mehrere Antioxidantien.

2. System nach Anspruch 1, **dadurch gekennzeichnet, dass** die Oberfläche gemäß (a) eine Oberfläche in einem Innenraum eines Gefäßes zum Aufbewahren einer oxidationsempfindlichen Substanz ist, und der Innenraum ferner das oder die Antioxidantien gemäß (b) enthält.

3. System nach einem der vorherigen Ansprüche, wobei die Oberfläche gemäß (a) herstellbar oder hergestellt ist durch Sintern des oder der Metalle und/oder Metalloxide auf eine Trägeroberfläche.

4. System nach einem der vorherigen Ansprüche, ferner umfassend als oxidationsempfindliche Substanz in Kontakt sowohl mit der Oberfläche gemäß (a) als auch mit dem oder den Antioxidantien gemäß (b) einen Riechstoff und/oder einen Aromastoff.

5. System nach einem der vorherigen Ansprüche, wobei die oxidationsempfindliche Substanz bei Oxidation an Luft eine α,β-ungesättigte Carbonylfunktion bildet.

6. System nach einem der vorherigen Ansprüche, wobei die oxidationsempfindliche Substanz ausgewählt ist aus der Gruppe bestehend aus Limonen, Geraniol und Citronellol.

7. System nach einem der vorherigen Ansprüche, enthaltend die oxidationsempfindliche Substanz in Form einer kosmetischen und/oder dermatologischen Zubereitung.

8. Verwendung eines Systems nach einem der vorherigen Ansprüche zum Schützen einer oxidationsempfindlichen Substanz, insbesondere eines Riechstoffs und/oder Aromastoffs, vor Oxidation.

9. Verwendung eines Systems nach einem der Ansprüche 1 bis 7 zum Unterdrücken der Ausbildung allergener Zersetzungsprodukte bei der Lagerung einer oxidationsempfindlichen Substanz.

10. Verwendung eines Systems nach einem der Ansprüche 1 bis 8 zum Verringern der Peroxidzahl bei der Lagerung einer oxidationsempfindlichen Substanz.

11. Verwendung nach einem der Ansprüche 8 bis 10, wobei die oxidationsempfindliche Substanz ausgewählt ist aus der Gruppe bestehend aus Limonen, Geraniol und Citronellol.
